# FASCICULE DE BREVET EUROPEEN

(11) **EP 1 113 984 B1**
(45) Date de publication et mention de la délivrance du brevet: **25.09.2002**
(21) Numéro de dépôt: 99929464.8
(22) Date de dépôt: 09.07.1999
(51) Int. Cl.: C01B 21/16

(54) **PROCEDE DE PREPARATION D'HYDRATE D'HYDRAZINE**
VERFAHREN ZUR HERSTELLUNG VON HYDRAZINHYDRAT
METHOD FOR PREPARING HYDRAZINE HYDRATE

(30) Priorité: 26.08.1998 FR 9810715
(43) Date de publication de la demande: 11.07.2001
(73) Titulaire: Atofina, 92800 Puteaux (FR)
(72) Inventeur: SCHIRMANN, Jean-Pierre, F-69600 Oullins (FR); BOURDAUDUCQ, Paul, F-69630 Chaponost (FR)
(74) Mandataire: Dang, Doris
(86) Numéro de dépôt international: FR9901685
(87) Numéro de publication internationale: WO00012430

(56) Documents cités:
- EP-A- 0 399 866
- EP-A- 0 758 642
- US-A- 4 657 751
- US-A- 5 252 309

## Description

La présente invention concerne un procédé de préparation d'hydrate d'hydrazine. La présente invention concerne plus précisément un procédé amélioré de fabrication d'hydrate d'hydrazine à partir d'azine de la méthyl éthyl cétone obtenue par oxydation de l'ammoniac par l'eau oxygénée en présence d'un coréactif ou d'un catalyseur.

La production industrielle de l'hydrate d'hydrazine se fait selon les procédés RASCHIG, BAYER ou à l'eau oxygénée.

Dans le procédé RASCHIG, on oxyde l'ammoniac par un hypochlorite pour obtenir une solution diluée d'hydrate d'hydrazine qu'il faut ensuite concentrer par distillation. Ce procédé peu sélectif, peu productif et très polluant, n'est presque plus utilisé.

Le procédé BAYER est une variante du procédé RASCHIG qui consiste à déplacer un équilibre chimique en piégeant, à l'aide d'acétone, l'hydrazine formée sous forme d'azine (CH₃)₂C=N-N=C-(CH₃)₂. L'azine est ensuite isolée puis hydrolysée en hydrate d'hydrazine. Les rendements sont améliorés, mais il n'y a pas d'amélioration quant aux rejets dans l'environnement.

Le procédé à l'eau oxygénée consiste à oxyder un mélange d'ammoniac et d'une cétone par l'eau oxygénée en présence d'un moyen d'activation de l'eau oxygénée pour faire directement l'azine qu'il suffit ensuite d'hydrolyser en hydrate d'hydrazine. Les rendements sont élevés et le procédé n'est pas polluant. Ce procédé à l'eau oxygénée est utilisé par la demanderesse et est décrit dans de nombreux brevets, par exemple US 3,972,878, US 3,972,876, US 3,948,902 et US 4,093,656.

L'hydrolyse d'une azine en hydrate d'hydrazine est décrite dans les brevets US 4 724 133 SCHIRMANN et al, US 4,725,421 SCHIRMANN et al et GB 1 164 460. Cette hydrolyse s'effectue dans une colonne de distillation qu'on alimente avec de l'eau et de l'azine, en tête on récupère la cétone et en pied l'hydrate d'hydrazine.

EP 70 155 décrit aussi un autre procédé à l'eau oxygénée.

Ces procédés sont aussi décrits dans ULLMANN'S ENCYCLOPEDIA OF INDUSTRIAL CHEMISTRY (1989), vol A 13, pages 182-183 et les références incluses.

Dans les procédés à l'eau oxygénée, on oxyde l'ammoniac par le peroxyde d'hydrogène en présence d'une cétone et d'un moyen d'activation du peroxyde d'hydrogène selon la réaction globale suivante, en faisant une azine :

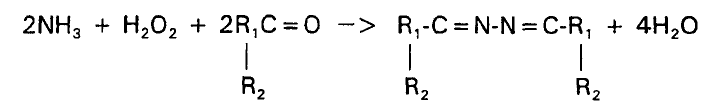

Le moyen d'activation peut être un nitrile, un amide, un acide carboxylique ou encore un dérivé du sélénium, de l'antimoine ou de l'arsenic. Puis l'azine est hydrolysée en hydrazine et la cétone régénérée est recyclée selon la réaction suivante :

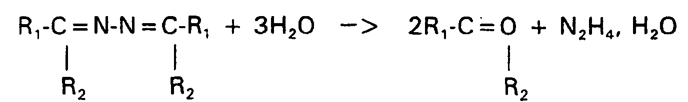

Cette hydrolyse s'effectue dans une colonne à distiller, on récupère la cétone en tête et l'hydrate d'hydrazine en pied.

Dans tous les procédés utilisant la MEK (méthyl éthyl cétone) et l'eau oxygénée, on constate la formation d'impuretés dérivées de la MEK et résultant de réactions secondaires. En particulier on constate la formation de butanol-2 qui provient d'une réduction de la méthyléthylcétone par un dérivé de l'hydrazine, peut être selon un processus du type diimine :

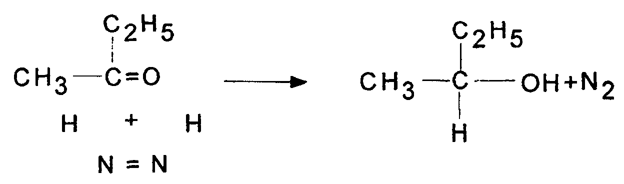

Cette formation de butanol-2 a déjà été décrite dans la demande de brevet européen EP 758 642 A2, et il est indiqué que le rendement en azine est considérablement détérioré si on laisse le butanol-2 s'accumuler et si son ratio par rapport à la méthyléthylcétone dépasse 0,05 mole par mole de MEK et qu'il était nécessaire de procéder à la séparation du butanol-2 par distillation puis à sa purge.

Dans la demande EP 758 642 il est utilisé un moyen d'activation de l'eau oxygénée constitué d'un mélange d'acide cacodylique (diméthyl arsenic) et d'acétate d'ammonium ou de propionate d'ammonium. Si la quantité de butanol-2 est de 0,05 mole par mole de MEK le rendement en azine par rapport à l'eau oxygénée est de 84 %. Si la quantité de butanol-2 est de 0,1 mole par mole de MEK le rendement en azine par rapport à l'eau oxygénée chute à 60 %. La demanderesse vient de découvrir que cet effet du butanol n'était pas général et était lié à la nature du système d'activation de l'eau oxygénée.

Ainsi la demanderesse vient de découvrir que contrairement à ce qui est décrit dans la demande de brevet EP 758 642, le butanol-2 n'a pas d'effet sur le rendement si le système d'activation du peroxyde d'hydrogène est constitué d'un mélange d'un amide d'acide carboxylique et du sel d'ammonium correspondant (par exemple acétamide et acétate d'ammonium) en solution aqueuse et qu'il n'y a pas de dérivé contenant de l'arsenic. On doit donc distinguer les systèmes d'activation pour lesquels le butanol a un effet sur le rendement en azine c'est-à-dire que le rendement en azine par rapport à l'eau oxygénée baisse quand le rapport butanol-2 / MEK augmente ou est supérieur à un certain seuil et les systèmes d'activation pour lesquels le butanol n'a aucun effet sur le rendement en azine.

La demanderesse a aussi découvert que dans les procédés utilisant un système d'activation pour lequel le butanol est sans effet sur le rendement, il est utile de purger ce butanol parce que bien qu'il ne fasse pas chuter le rendement de la réaction comme décrit plus haut, il prend la place de la MEK et la production baisse par manque de réactif. On purge donc le butanol-2 de façon à ce que sa proportion molaire par rapport à la MEK soit par exemple de 5 à 15 moles pour 100 moles de MEK. Ainsi cette purification de la MEK est facile parce que cette teneur n'est pas trop basse et est facile à atteindre et on peut accepter des fluctuations sur le niveau de butanol-2 dans la MEK sans qu'il y ait de variations sur le rendement de la réaction de synthèse d'azines.

La présente invention est donc un procédé de préparation d'azine de la méthyl éthyl cétone dans lequel :
(a) on fait réagir l'ammoniac, le peroxyde d'hydrogène et la méthyl éthyl cétone en présence d'une solution de travail comprenant un système d'activation constitué d'un mélange d'un amide d'acide carbonylique et du sel d'ammonium correspondant pour former une azine ;
(b) on sépare la solution de travail et l'azine contenant éventuellement la méthyl éthyl cétone n'ayant pas réagi et du butanol-2 ;
(c) on recycle la solution de travail à l'étape (a) après un traitement éventuel ;
(d) on sépare la méthyl éthyl cétone et le butanol-2 d'avec l'azine ;
(e) on purge une partie du butanol-2 du courant de l'étape (d) de façon à maintenir entre 0,05 et 0,15 le rapport molaire butanol-2 / MEK à l'entrée de l'étape (a).

Dans un mode d'exécution préféré l'azine recueillie à l'étape (d) est hydrolysée dans une étape (f) pour obtenir de l'hydrate d'hydrazine, la méthyl éthyl cétone régénérée est recyclée à l'étape (a).

### Etape (a)

L'eau oxygénée peut être utilisée sous la forme commerciale habituelle, par exemple en solution aqueuse entre 30 et 90 % en poids de H₂O₂. Avantageusement on peut ajouter un ou plusieurs stabilisants usuels des solutions peroxydiques, par exemple de l'acide phosphorique, pyrophosphorique, citrique, nitrilo-triacétique, éthylènediaminetétracétique ou les sels d'ammonium ou de métal alcalin de ces acides. La quantité à utiliser est avantageusement comprise entre 10 et 1 000 ppm et, de préférence, entre 50 et 250 ppm de l'ensemble des réactifs et de la solution de travail à l'entrée du réacteur. L'ammoniac peut être anhydre ou en solution aqueuse.

La solution de travail peut être aqueuse ou à base d'un alcool ou d'un mélange d'alcool et eau. Parmi les alcools, on utilise avantageusement les alcools aliphatiques saturés ayant de 1 à 6 atomes de carbone et, de préférence, 1 à 2 atomes de carbone.

On utilise aussi avantageusement des diols et plus particulièrement des diols ayant de 2 à 5 atomes de carbone. On peut citer par exemple le glycol, le propylèneglycol, le 1,3 propanediol, le 1,3 et 1,4 butanediol et le 1,5 pentanediol.

Les réactifs peuvent être utilisés en quantités stoechiométriques, cependant on utilise par mole d'eau oxygénée 0,2 à 5 moles et de préférence 1,5 à 4 moles de méthyl éthyl cétone ; 0,1 à 10 moles et de préférence 1,5 à 4 moles d'ammoniac. La quantité de solution de travail est comprise entre 0,1 et 1 kg par mole d'eau oxygénée. Cette quantité dépend de sa qualité, c'est-à-dire de sa force catalytique ou son activité qui permet de convertir les réactifs en azine. Les proportions des réactifs fixées ci-dessus permettent d'obtenir une conversion totale de l'eau oxygénée et une production d'azine correspondant à plus de 50 % de l'eau oxygénée engagée et pouvant atteindre 90 %.

La mise en contact de l'eau oxygénée, de l'ammoniac, de la méthyl éthyl cétone avec la solution de travail peut s'effectuer de façon quelconque.

Avantageusement, on opère dans un milieu homogène ou dans un milieu qui assure au moins une solubilisation suffisante des réactifs pour pouvoir obtenir de l'azine. La réaction peut se faire dans une très large plage de température, par exemple entre 0 et 100°C, et on opère avantageusement entre 30 et 70°C. Bien qu'on puisse opérer à toute pression, il est plus simple d'être à la pression atmosphérique, mais on peut monter jusqu'à environ 10 bars si c'est nécessaire pour maintenir de préférence la réaction de l'étape a en phase liquide.

Les réactifs peuvent être introduits simultanément ou séparément et dans un ordre quelconque dans la solution de travail. On peut utiliser toutes sortes de réacteurs, agités ou non agités, ou même de simples capacités qu'on peut disposer en parallèle, en série, à co-courant ou à contre-courant, ou tout combinaison de ces possibilités.

### Etape (b)

On sépare (i) l'azine, l'excès de méthyl éthyl cétone et le butanol-2 d'avec (ii) la solution de travail par des moyens connus tels que l'extraction liquide-liquide, la distillation, la décantation ou toute combinaison de ces possibilités.

La méthyl éthyl cétone est avantageuse parce que son azine est insoluble dans la solution de travail.

Dans l'étape (c), la solution de travail peut être traitée.

Les étapes (a), (b) et (c) sont décrites par exemple dans les brevets EP 399 866 et EP 518 728 dont le contenu est incorporé dans la présente demande.

### Etape (d)

On sépare la méthyl éthyl cétone et le butanol-2 d'avec l'azine. on peut opérer par distillation à pression atmosphérique ou sous pression réduite. L'azine de la méthyl éthyl cétone (désignée aussi par mekazine) est recueillie en pied de la colonne de distillation.

L'étape (f) s'effectue par exemple dans une colonne à plateaux ou à garnissage de type colonne de distillation qu'on alimente avec l'azine provenant de l'étape (b) et de l'eau. On obtient (i) en tête, la méthyl éthyl cétone sous forme d'un azéotrope avec l'eau et (ii) en pied, une solution aqueuse d'hydrate d'hydrazine.

L'hydrolyse des azines est connue. Par exemple, E.C. GILBERT, dans un article dans le Journal of American Chemical Society vol.51, p. 3397-3409 (1929), décrit les réactions équilibrées de formation d'azine et les réactions d'hydrolyse de celles-ci et fournit les paramètres thermodynamiques du système dans le cas d'azines solubles dans l'eau. Par exemple, l'hydrolyse de l'azine de l'acétone est décrite dans US 4,724,133. S'agissant des azines insolubles dans les solutions aqueuses (par exemple l'azine de la méthyl éthyl cétone 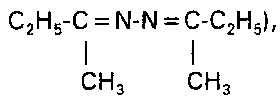 l'hydrolyse doit être réalisée dans une colonne réactive, de telle sorte qu'en séparant continuellement la méthyl éthyl cétone en tête de colonne de distillation et l'hydrate d'hydrazine en pied de colonne, on peut parvenir à une hydrolyse totale. Bien entendu ce système est au meilleur de son fonctionnement lorsqu'on travaille en continu comme décrit dans les brevets français 1 315 348 ou anglais 1 211 547, ou encore le brevet US 4,725,421.

Dans tous ces brevets, la réaction est réalisée dans une colonne de distillation à garnissage ou mieux à plateaux fonctionnant sous une pression de 2 à 25 bars et avec une température de pied de 150°C à 200°C.

Lorsqu'on travaille avec de l'azine pure, c'est-à-dire obtenue par exemple à partir d'hydrate d'hydrazine et de méthyl éthyl cétone, on constate effectivement en travaillant selon ces brevets, que l'on obtient avec un bon rendement des solutions diluées d'hydrate d'hydrazine.

Dans cette colonne, s'effectue l'hydrolyse de l'azine et la séparation de l'hydrate d'hydrazine d'avec la méthyl éthyl cétone. Ces conditions sont connues. L'homme de métier détermine facilement le nombre de plateaux ou la hauteur de garnissage, ainsi que les points d'alimentation en azine et en eau. On obtient en pied des solutions à 30 ou même jusqu'à 45 % en poids d'hydrate d'hydrazine. Ce rapport molaire eau/azine à l'alimentation de cette colonne est au moins supérieur à la stoechiométrie et avantageusement compris entre 5 et 8. Le pied de colonne est entre 150 et 200°C, de préférence 175 à 190°C. La pression est fonction de la température d'ébullition de l'azine, de l'eau et du réactif portant un groupe carbonyle. Une telle hydrolyse est décrite dans US 4,725,721.

La purge de butanol-2 de l'étape (e) sert à éviter son accumulation. Puisqu'on ne déshydrogène pas ce butanol-2 pour le recycler dans l'étape (a) il est préférable de purger un courant de butanol-2 aussi pur que possible pour éviter de perdre d'autres produits en particulier de la MEK.

### Exemple 1 (non conforme à l'invention)

On charge dans un réacteur 240 g d'eau (13,3 moles), 118 g de propionate d'ammonium (1,3 mole) ainsi que 40 g d'acide cacodylique (0,29 mole) et 2 cm³ de stabilisant Dequest 2066 (sel de sodium de l'acide diethylène diamine penta (méthylène phosphonique) en solution aqueuse à 25 %) vendu par la Société MONSANTO. On porte ce mélange à 55°C, puis on introduit de l'ammoniac gazeux jusqu'à saturation. On ajoute alors en une heure 38,9 g d'eau oxygénée 70 % (0,8 mole) ainsi que 144 g de méthyléthylcétone contenant 2 % molaire de butanol-2 (2 moles). On maintient encore sous agitation pendant deux heures à 55°C en continuant à introduire de l'ammoniac gazeux. Puis on laisse refroidir. On sépare les phases organique et aqueuse et on les analyse. On constate que la conversion de l'eau oxygénée est de 65,4 % et que le rendement en azine est de 45,8 %.

### Exemple 2 (non conforme à l'invention)

On répète l'exemple 1 mais on remplace le propionate d'ammonium par 100 g d'acétate d'ammonium (1,3 mole) et on laisse la réaction durer 7 heures à 55°C. La conversion de l'eau oxygénée est de 79,3 % et le rendement en azine de la méthyléthylcétone de 54 %.

### Exemple 3 (non conforme à l'invention)

On répète l'exemple 2 en utilisant de la méthyléthylcétone contenant 10 % molaire de butanol-2. La conversion de l'eau oxygénée est de 79,5 % alors que le rendement en azine chute à 39,8 %.

### Exemple 4 (conforme à l'invention)

On charge dans un réacteur 180 g d'eau (10 moles), 77 g d'acétate d'ammonium (1 mole), 177 g d'acétamide (3 moles), 144 g de méthyléthylcétone (2 moles) et 2 cm³ de stabilisant Dequest 2066 (sel de sodium de l'acide diethylène diamine penta (méthylène phosphonique) en solution aqueuse à 25 %) vendu par la Société MONSANTO. On porte ce mélange à 50°C, puis on sature en ammoniac. On ajoute alors 48,5 g de peroxyde d'hydrogène 70 % (1 mole) sur une durée de 1 heure. On continue à introduire de l'ammoniac gazeux de façon à maintenir le milieu saturé. On laisse réagir 7 heures à 50°C. Après refroidissement à température ordinaire, on sépare les phases aqueuse et organique. On analyse et trouve que la conversion de H₂O₂ est de 98 % pour un rendement en azine de 81 %.

### Exemple 5 (conforme à l'invention)

On répète l'exemple 4, mais en utilisant de la méthyléthylcétone contenant 10 % molaire de butanol-2. La conversion du peroxyde d'hydrogène est de 99 % et le rendement en azine de 81,9 %.

## Revendications

1. Procédé de préparation d'azine de la méthyl éthyl cétone dans lequel :
a) on fait réagir l'ammoniac, le peroxyde d'hydrogène et la méthyl éthyl cétone en présence d'une solution de travail, comprenant un système d'activation constitué d'un mélange d'un amide d'acide carboxylique et du sel d'ammonium correspondant pour former une azine ;
b) on sépare la solution de travail et l'azine contenant éventuellement la méthyl éthyl cétone n'ayant pas réagi et du butanol-2 ;
c) on recycle la solution de travail à l'étape (a) après un traitement éventuel ;
d) on sépare la méthyl éthyl cétone et le butanol-2 d'avec l'azine ;
e) on purge une partie du butanol-2 du courant de l'étape (d) de façon à maintenir entre 0,05 et 0,15 le rapport molaire butanol-2 / MEK à l'entrée de l'étape (a).

2. Procédé selon la revendication 1 dans lequel l'azine recueillie à l'étape (d) est hydrolysée dans une étape (f) pour obtenir de l'hydrate d'hydrazine, la méthyl éthyl cétone régénérée est recyclée à l'étape (a).

## Patentansprüche

1. Verfahren zur Herstellung des Azins von Methylethylketon mit den folgenden Schritten:
a) Umsetzung von Ammoniak, Wasserstoffperoxid und Methylethylketon in Gegenwart einer Arbeitslösung, die ein Aktivatorsystem enthält, das aus einem Gemisch eines Amids einer Carbonsäure und des entsprechenden Ammoniumsalzes besteht, zur Bildung eines Azins;
b) Trennung der Arbeitslösung und des Azins, das gegebenenfalls Methylethylketon, das nicht umgesetzt wurde, und 2-Butanol enthält;
c) Rückführung der Arbeitslösung zu Schritt a) nach einer gegebenenfalls durchgeführten Behandlung;
d) Abtrennung von Methylethylketon und 2-Butanol von dem Azin;
e) teilweise Entnahme von 2-Butanol aus dem Strom von Schritt d), um das Molverhältnis 2-Butanol/MEK am Beginn von Schritt a) im Bereich von 0,05 bis 0,15 zu haltend.

2. Verfahren nach Anspruch 1, wobei das in Schritt d) gewonnene Azin in einem Schritt f) hydrolysiert wird, um Hydrazinhydrat herzustellen, wobei das wiedergewonnene Methylethylketon zu Schritt a) rückgeführt wird.

## Claims

1. Process for the preparation of methyl ethyl ketone azine, in which:
(a) ammonia, hydrogen peroxide and methyl ethyl ketone are reacted in the presence of a working solution, comprising an activating system composed of a mixture of a carboxylic acid amide and of the corresponding ammonium salt, in order to form an azine;
(b) the working solution and the azine, optionally comprising unreacted methyl ethyl ketone and 2-butanol, are separated;
(c) the working solution is recycled to the stage (a) after an optional treatment;
(d) the methyl ethyl ketone and the 2-butanol are separated from the azine;
(e) part of the 2-butanol is bled off from the stream from the stage (d), so as to maintain the 2-butanol/MEK molar ratio between 0.05 and 0.15 at the inlet of the stage (a).

2. Process according to Claim 1, in which the azine collected in the stage (d) is hydrolysed in a stage (f) in order to obtain hydrazine hydrate and the regenerated methyl ethyl ketone is recycled to the stage (a).
